## Europäisches Patentamt

## European Patent Office

## Office européen des brevets

(11) Publication number: **0 150 943**
**B1**

## (12) EUROPEAN PATENT SPECIFICATION

(45) Date of publication of patent specification: **04.01.89**

(51) Int. Cl.⁴: **C 07 D 307/20, B 01 J 31/24, B 01 J 31/28**

(21) Application number: **85300219.4**

(22) Date of filing: **11.01.85**

(54) Hydroformylation of allyl alcohol.

(30) Priority: **02.02.84 US 576509**

(43) Date of publication of application:
**07.08.85 Bulletin 85/32**

(45) Publication of the grant of the patent:
**04.01.89 Bulletin 89/01**

(84) Designated Contracting States:
**BE DE FR GB NL**

(56) References cited:
EP-A-0 038 609
DE-A-2 649 900
US-A-4 064 145
US-A-4 139 542
US-A-4 400 548

JOURNAL OF ORGANIC CHEMISTRY, vol. 45,
no. 11, May 1980, pages 2132-2139, Easton,
US; C.U. PITTMAN et al.: "Rhodium-catalyzed
hydroformylation of allyl alcohol. A potential
route to 1,4-Butanediol"

(73) Proprietor: **TEXACO DEVELOPMENT
CORPORATION**
**2000 Westchester Avenue**
**White Plains New York 10650 (US)**

(72) Inventor: **Lin, Jiang-Jen**
**2617 Oak Meadow Drive**
**Round Rock Texas 78664 (US)**
Inventor: **Knifton, John Frederick**
**10900 Catskill Trail**
**Austin Texas 78750 (US)**

(74) Representative: **Burnside, Michael et al**
**Urquhart-Dykes & Lord 91 Wimpole Street**
**London W1M 8AH (GB)**

(56) References cited:
CHEMIKER-ZEITUNG, vol. 101, nos. 7/8, 1977,
pages 343-350; B. FELL et al.:
"Isomerisierungsfreie Hydroformylierung mit
Rhodiumcarbonyl/tert. Phosphin-
Komplexkatalysatoren, II. Die
Hydroformylierung von Allylalkoholen"

Courier Press, Leamington Spa, England.

# EP 0 150 943 B1

**Description**

This invention is related to the addition of hydrogen and carbon monoxide to olefin compounds to obtain hydroxy-substituted cyclic compounds in the presence of a rhodium-containing catalyst and is more particularly related to such an addition conducted in the presence of a ketone solvent whereby allyl alcohol is converted into 2-hydroxytetrahydrofuran.

2-hydroxytetrahydrofuran is an important intermediate for producing 1,4-butanediol. A number of methods have been discovered for hydroformylating various unsaturated compounds to useful products.

DE—A—2 649 900 discloses a process for the preparation of 2-hydroxytetrahydrofuran by reacting allyl alcohol with carbon monoxide and hydrogen in the presence of Rh $HCO(PPh_3)_3$, an organic phosphine and a solvent such as toluene or benzene, whereby a maximum conversion of 86% and a maximum selectivity of 84% are obtained.

In accordance with EP—A—0 038 609, the conversion of allyl alcohol with hydrogen and carbon monoxide in the presence of e.g. Rh $HCO(PPh_3)_3$ and phosphine is performed continuously in the gas phase whereby a conversion of about 20% and a maximum selectivity of 97.8% are reached.

US—A—4 209 467 teaches a low pressure hydroformylation process in which the catalyst is a reaction product of a cobalt carbonyl and a nitrogen-containing heterocyclic compound having an enolic hydroxyl group on the carbon atom adjacent to the ring-forming nitrogen atom, e.g. 2-hydroxypyridine. Ordinarily, the pressures employed therein are in the neighbourhood of 1 to 10 MPa (10 to 100 atmospheres). Unsaturated compounds taught as suitable for this hydroformylation process include ethylenically unsaturated hydrocarbons such as ethylene, propylene, butadiene, and other ethylenically unsaturated compounds such as allyl alcohol and allyl acetate.

Rhodium catalysts are also known for such hydroformylation processes. US—A—3 980 670 discloses a process for manufacturing methacrylic acid and butyrolactone by hydroformylation of allyl esters of lower carboxylic acids in the presence of rhodium carbonyl complex catalysts, followed by oxidation of the resulting formyl compounds with molecular oxygen to produce 4-acetoxy-n-butyric acid and 3-acetoxy-isobutyric acid as the major products, see also DE—A—2 106 243. Unsaturated compounds, such as propylene, may be hydroformylated by means of rhodium/triphenylphosphine/carbonyl complexes formed in situ using a special pre-forming step described in US—A—4 400 549.

US—A—4 064 145 and —4 083 882 described a method for producing tetrahydrofuran and 1,4-butanediol by reacting synthesis gas with allyl alcohol under hydroformylation conditions in the presence of a rhodium carbonylphosphine catalyst complex and various inert solvents such as aromatic and aliphatic hydroxylic solvents. In both patents, the allyl alcohol conversion was reported to be 99% and 4 - hydroxybutanal was typically obtained in 87 wt% yield. The major by-product was 2 - methyl - 3 - hydroxypropanol (12 wt%). A rhodium catalyst complexed with special bisphosphine mono-oxide ligands is taught as catalyzing the hydroformylation of olefinic compounds in the presence of dimethylformamide solvent, according to US—A—4 400 548.

In *J. Org. Chem. 45* (1980), 2132, C.U. Pittman, Jr. disclosed the hydroformylation of allyl alcohol to 4 - hydroxybutanal and 3 - hydroxy - 2 - methylpropanal using $HRh(CO)(PPh_3)_3$ and polymer-bound analogues thereof. The selectivity towards normal/branched products was studied as a function of reaction parameters and ligands employed. The highest normal/branched selectivities (80%) were reported with 1,1' - bis(diphenylphosphino)ferrocene. Benzene and o-xylene were generally used as solvents.

In *J. of Mol. Cat., 11,* (1981) 233—246, N.A. deMunck reported in a heterogeneous gas phase hydroformylation of allyl alcohol using a supported $HRh(CO)(PPH_3)_3$ catalyst. A very high selectivity to 4 - hydroxybutyraldehyde (97%) was achieved, but the conversion of allyl alcohol was only about 20%.

JP—A—29412/1976 and 106407/1979, and *Chemical Economy and Engineering Review*, Vol. 12, No. 9, (1980) disclose the hydroformylation of allyl alcohol using rhodium catalysts in organic solvents, such as benzene and toluene and a diphosphinoalkane. The overall n-/iso-ratio of the products was 86.6/13.4. In JP—A—84508/1979 and GB—A—1 493 154 a modified Raney catalyst was employed for the hydrogenation of hydroxybutyraldehydes, forming 1,4-butanediol and 3-methyl-1,3-butanediol.

Many of the systems described above lack good conversions of the unsaturated reactant compound and/or good selectivity to the desired product. Further, stability of expensive rhodium catalyst is a problem in many of these processes. It would be an advance in the art if a method could be devised for hydroformylating compounds such as allyl alcohol while simultaneously solving the conversion selectivity and catalyst stability problems noted above.

The invention concerns a process in which 2 - hydroxytetrahydrofuran is obtained by hydroformylating allyl alcohol by reaction with carbon monoxide and hydrogen at a temperature above 25°C and a pressure above 0.75 MPa. A rhodium catalyst and a ketone solvent catalyst system are employed.

In general, the components of the hydroformylation reaction mixture, including the inert solvents, allyl alcohol and rhodium catalyst, may be added in any sequence as long as good agitation is employed to provide a good dispersion or a homogeneous reaction mixture. For example, the following represent some variations in the addition of catalyst components, inert solvents and allyl alcohol addition that can be made without departing from the inventive process. These modifications include:

2

1. The catalyst may be performed and added to the reaction solvents before addition of the allyl alcohol and other inert solvent components.

2. Alternatively, to minimize catalyst-stability problems, the catalyst can be formed in situ, usually by mixing the inert solvents and allyl alcohol, followed by the addition of the catalyst components to form the reaction mixture.

3. After using either variation 1 or 2, the deoxygenated catalyst-containing reaction mixture is pressurized with CO and hydrogen and heated until the hydroxy tetrahydrofuran product is formed.

A rhodium catalyst is used in the present invention. Any rhodium-containing compound capable of forming a carbonyl under the reaction conditions can be used. This rhodium compound may be a carbonyl such as hexarhodium hexadecacarbonyl. Preferably, the rodium carbonyl is complexed with a phosphine ligand. Such catalysts are described in US—A—4 064 145; 4 400 548 and 4 400 549. It is especially preferred that the catalyst be a rhodium carbonyl triphenylphosphine complex catalyst such as hydridocarbonyl-tris(triphenylphosphine)rhodium (I), $HRh(CO)(PPh_3)_3$, where Ph represents phenyl. Preferably, an excess of the phosphine ligand is added to provide additional triphenylphosphine.

As noted, the novel feature of the invention is the solvent system. The solvent should be a ketone. Useful ketones may contain straight or branched aliphatic araliphatic groups, cycloaliphatic groups or aromatic groups. Suitable ketones include methyl isobutyl ketone 2 - decanone, 4 - decanone, benzylacetone, di - $n$ - hexyl ketone, 1,3 - diphenylacetone, methyl ethyl ketone, acetone, 2,6 - dimethyl - 4 - heptanone, 2 - undecanone, 3 - undecanone, benzophenone, 2 - methylbenzophenone, 3 - methylbenzophenone, substituted, benzophenones, n-butyrophenone, acetophenone, ortho-, para- and meta-substituted acetophenones, such as p - ethylacetophenone and o - methoxyacetophenone, and phenyl ethyl ketone. The ketone solvents should be inert with respect to rhodium-catalyzed carbonylation and reduction reactions.

The preferred ketone solvents for rhodium-catalyzed hydroformulation of allyl alcohol have been found to be either 2-undecanone or acetophenone. It has been surprisingly discovered that the use of these solvent-catalyst combinations increases the allyl alcohol conversion and selectivity to 2-hydroxytetrahydrofuran and also increases the rhodium catalyst stability.

The temperature range which can be employed for hydroformylation is a variable which is dependent upon experimental factors including the total pressure, the mole ratio of hydrogen and carbon monoxide used, and the concentrations of reactants and catalyst, among other things. Using rhodium carbonyltriphenylphosphine complex as a representative catalyst, an operable range is from 25 to 125°C, and even higher, when superatmospheric pressures above 0.75 MPa are employed. A narrower range of 50 to 120°C represents the preferred temperature range when allyl alcohol is hydroformylated.

The pressure range which can be employed for hydroformylation is a variable which is also dependent on the factors mentioned above. Using rhodium carbonyltriphenylphosphine as a representative catalyst, an operable pressure range is from 0.75 to 35 MPa, or even higher with a mol ratio $H_2/CO$ of 1:1 when a temperature range of from 25 to 125°C is employed. A narrower range of from 3.5 to 10.5 MPa represents the preferred pressure range when the narrower temperature range of 50 to 125°C is employed.

The $H_2/CO$ mol ratio may be varied over a range of from 30:1 to 1:30 when suitable temperatures and pressures are employed. A preferred narrower range of hydrogen/carbon monoxide is from 2:1 to 1:2.

Experimental variables are important in arriving at reaction times. Generally, substantial conversions (90% or higher) of the allyl alcohol to 2-hydroxytetrahydrofuran can almost always be accomplished within 18 hours, with 2 to 6 hours representing the more usual reaction time interval.

Experimental work indicates that an initial mol ratio of 10 to 10,000 mols of allyl alcohols per mol of rhodium-containing catalyst complex can be employed in most instances. The minimum ratio of 0.0001 mols of catalyst per mol of allyl alcohol is herein referred to as a "catalytic ratio" or "catalytic amount". Much higher ratios (e.g. 25 mols of substrate per mol of rhodium catalyst complex) are not harmful, but are economically unattractive. For this reason the favoured mol ratio ranges from 50 to 5,000 mols of allyl alcohol per mol of rhodium catalyst complex.

The most particularly preferred combination of reaction conditions include a synthesis gas mol ratio (carbon monoxide to hydrogen) of about 1:1, a mol ratio of rhodium carbonyl-triphenylphosphine complex catalyst to excess triphenylphosphine of about 1:100, a temperature of from 60 to 80°C and an operating pressure of 5.0 to 6.5 MPa. Again, the ketone solvent should preferably be either acetophenone or 2-undecanone. It was surprisingly discovered that when this unique combination of parameters was used, the selectivity to 2-hydroxytetrahydrofuran is at least 96% and the allyl alcohol conversion is about 100%. Results this good have not been obtained by any previous method.

The hydroformylation product, 2-hydroxytetrahydrofuran, may be isolated by the usual chemical or physical techniques, such as distillation, solvent extraction, or chromatography. Identification can be by nuclear magnetic resonance and/or gas-liquid chromatography.

Conversion as defined herein represents the extent to which allyl alcohol is converted into other products. Conversion is expressed as a percentage and is calculated by dividing the amount of allyl alcohol consumed during hydroformylation by the amount of alcohol originally charged and multiplying the quotient by 100. The allyl alcohol conversion in the process of this invention can be at least 90%.

Yield, as defined herein, represents the efficiency in catalyzing the desired hydroformylation reaction relative to other undesired reactions. In this instance hydroformylation to 2 - hydroxytetrahydrofuran is the desired conversion. Yield is expressed as a percentage and is the amount of 2 - hydroxytetrahydrofuran product formed, divided by the amount of allyl alcohol charged, the quotient being multiplied by 100.

Selectivity, as defined herein, is the efficiency in catalyzing a desired hydroformylation reaction relative to all other (undesired) conversion. Selectivity is expressed as a percentage and is calculated by dividing the amount of 2 - hydroxytetrahydrofuran product formed, by the total amount of aliphatic products formed, and multiplying the quotient by 100. Selectivity can be at least 90% for the process according to the invention.

Experimental Example A

A 300 ml stainless steel stirred autoclave was charged with HRh(CO)(PPh$_3$)$_3$ (0.046 g, 0.05 mmol), Ph$_3$P (1.3 g, 50 mmol), allyl alcohol (10.0 g, 172 mmol) and acetophenone (10.0 g). The reactor was purged of air and pressurized to 0.79 MPa with a mixture of carbon monoxide and hydrogen at a mol ratio of 1:1, and then heated to 60°C. The pressure was brought to 5.61 MPa and maintained by the addition of carbon monoxide and hydrogen (1:1 molar ratio) from a supply gas cylinder. Aliquot samples were taken through a sample valve at the designated reaction time. After 4 hours, the reaction was stopped and the reactor was allowed to cool to room temperature. The excess gas was vented from the reactor following which 23.3 g light yellow solution was recovered.

Samples of liquid products were analyzed by gas-liquid chromatography and the product selectivities were estimated to be:

| Samples | Allyl alcohol conversion (%) | 2-hydroxytetra-hydrofuran selectivity (%) |
|---|---|---|
| 1st (1 hr) | >82 | 96 |
| 2nd (2 hr) | 100 | 96 |
| 3rd (4 hr) | 100 | 96 |

The typical by-product mixtures (at about 4% selectivity) were n-propanol, isobutyraldehyde, methacrolein and n-propanal. The remaining liquid product from the experiment, after completion of the analytical testing, was subjected to distillation under high vacuum (about 2.67 Pa) and the distillates were collected at <50°C boiling point.

The residual catalyst solid (pale yellow, 1.5 g) was then dissolved and combined with fresh allyl alcohol (10.0 g) and acetophenone (10.0 g) and charged to the autoclave. The identical experimental procedures were repeated in the second catalyst cycle, the following results obtained:

| Sample | Allyl alcohol conversion (%) | 2-hydroxytetra-hydrofuran selectivity (%) |
|---|---|---|
| 1st (1 hr) | 70 | >96 |
| 2nd (2 hr) | 93 | >96 |
| 3rd (4 hr) | >96 | >96 |

In the third catalyst cycle under the above reaction conditions the results were:

| Sample | Allyl alcohol conversion (%) | 2-hydroxytetra-hydrofuran selectivity (%) |
|---|---|---|
| 1st (1 hr) | 69 | >96 |
| 2nd (2 hr) | 93 | >96 |
| 3rd (4 hr) | 96 | >96 |

Thus, the stability of the catalyst in a recycle mode was confirmed.

Examples B—E

In these studies on the hydroformylation of allyl alcohol catalyzed by HRh(CO)(PPh$_3$)$_3$, the purpose is to focus on determining the effect of solvent structure upon allyl alcohol conversion, selectivity to 2-hydroxytetrahydrofuran and catalyst stability. The experimental procedure is that of Example A. The allyl alcohol charge at the beginning of each run was 10.0 g, the quantities of rhodium catalyst, phosphine ligand and added solvent are given in Table I.

TABLE I
Solvent effect on hydroformylation of allyl alcohol

| Example | Catalyst | Ligand | Solvent | Syngas mol ratio, $(CO/H_2)$ | Conditions | Liq-recovery, (g) | Allyl alcohol Conv. (%) | Selectivities (A%) | | |
|---------|----------|--------|---------|---------------------------------|------------|-------------------|------------------------|--------------------------------|-----------------|------------------------|
| | | | | | | | | 2-hydroxy-tetrahydro-furan | 1,4-Butane-diol | $C_3$ By-product |
| B | $HRh(CO)(PPh_3)_3$ (0.046 g) | $Ph_3P$ (1.3 g) | None | 1:2 | 5.61 Mpa 69—82°C 5 hrs. | 11.7 | 85 | 31 | 0 | 34 |
| C | " " | $Ph_3P$ (1.3 g) | 2-undecanone (10 g) | 1:2 | 5.61 MPa 73—83°C 4 hrs | 24.7 | >95 | 68 | 0 | 32 |
| D | " " | $Ph_3P$ (1.3 g) | 2-undecanone 10 g)+ 1,4-butanediol (7.7 g) | 1:2 | 5.61 MPa 77—80°C 5 hrs | 30.8 | >95 | 60 | — | 40 |
| E | " " | $Ph_3P$ (1.3 g) | 2-undecanone | 1:1 | 5.61 MPa 60°C 1 hr | — | 86 | 92 | 0 | 4 |

EP 0 150 943 B1

A number of observations may be made about these experimental runs. For example, a comparison of Examples B and C reveals that the addition of 2-undecanone solvent increases the allyl alcohol conversion and selectivity to 2-hydroxytetrahydrofuran. Comparison of Examples C and E teaches that the selectivity to 2-hydroxytetrahydrofuran is sensitive to the composition of synthesis gas feed; a mol ratio of CO to $H_2$ of 1:1 is preferred (see Example E). In Example D, the addition of 1,4-butanediol to the feed mixture does not seriously inhibit the formation of 2-hydroxytetrahydrofuran.

At the optimal reaction conditions of 5.61 MPa, a $CO/H_2$ mol ratio of 1:1, 60°C, reaction time of 2 to 4 hours and using a $HRh(CO)(PPh_3)_3/Ph_3P$ mol ratio of 1:100 in ketone solvents, a 96% selectivity to 2-hydroxytetrahydrofuran and a 100% allyl alcohol conversion is achieved; e.g., Example A.

Example F
Catalyst recycles in various solvents
Reaction conditions: $CO/H_2=1:2$; 5.61 MPa; 2 hours, 60°C
Catalyst: $HRh(CO)(PPh_3)_3$ (0.092 g) and triphenylphosphine 2.6 g)
Materials charged: Allyl alcohol (20 g) and solvent (20 g)

TABLE II
Conversion of allyl alcohol (%)

| Cycles | in 2-undecanone | p-xylene | n-Pr$_3$N |
|--------|-----------------|----------|-----------|
| 1st | >55 | >50 | >95 |
| 2nd | 100 | 90 | 55 |
| 3rd | 100 | 57 | 54 |

Example G
Catalyst recycles in various solvents
Reaction conditions: $CO/H_2=1:1$; 5.61 MPa; 60°C
Catalyst: $HRh(CO)(PPh_3)_3$ (0.046 g) and triphenylphosphine (1.3 g)
Materials charged: Allyl alcohol (10.0 g) and solvent (10.0 g)

TABLE III

| Cycles | Sample time | in p-xylene | | in acetophenone | |
|--------|-------------|-------------|-------------|-----------------|-------------|
| | | Conversion % | Selectivity % | Conversion % | Selectivity % |
| I | 2 hrs. | 100 | 94 | 100 | 96 |
| II | 2 hrs. | 88 | >96 | >93 | >96 |
| | 4 hrs. | 100 | 96 | 100 | 96 |
| III | 2 hrs. | 89 | >96 | 93 | >96 |
| | 4 hrs. | 100 | 96 | 100 | 96 |

Comparative results presented in Tables II and III show the importance of solvent structure with respect to the stability of the rhodium catalysts. Acetophenone and 2-undecanone are found to be superior. It is interesting to note that the residue of each distillation contained no significant amount of heavy materials, indicating the stability of 2-hydroxytetrahydrofuran during distillation.

Many modifications may be made by one skilled in the art without departing from the spirit and scope of the invention which are defined only by the appended claims. For example, solvents, proportions and reaction conditions could be changed to optimize the yield of 2-hydroxytetrahydrofuran.

**Claims**

1. A process for the hydroformylation of allyl alcohol, by reacting allyl alchol with carbon monoxide and hydrogen at a temperature above 25°C and a pressure above 0.75 MPa in the presence of a catalyst comprising a rhodium carbonyl or a rhodium compound capable of forming a rhodium carbonyl in the presence of carbon monoxide, and in the presence of a solvent, characterized in that the solvent is a ketone whereby the hydroformylation reaction produces 2-hydroxy tetrahydrofuran.

2. A process according to Claim 1 characterized in that the ketone solvent is acetophenone or 2-undecanone.

3. A process according to Claim 1 or 2 characterized in that the temperature is from 50 to 120°C and the pressure is from 3.5 to 10.5 MPa.

4. A process according to any of Claims 1 to 3 characterized in that the catalyst is a rhodium carbonyl-triphenylphosphine complex together with excess triphenylphosphine.

5. A process according to any of Claims 1 to 3 characterized in that the rhodium carbonyl catalyst is hydridocarbonyltris(triphenylphosphine)rhodium (I) in the presence of excess triphenylphosphine.

**Patentansprüche**

1. Verfahren zur Hydroformylierung von Allylalkohol durch Umsetzung von Allylalkohol mit Kohlenmonoxid und Wasserstoff bei einer Temperatur über 25°C und einem Druck über 0,75 MPa in Gegenwart eines Katalysators enthaltend oder bestehend aus einem Rhodium-carbonyl oder einer Rhodium-Verbindung, die zur Bildung eines Rhodiumcarbonyls in Gegenwart von Kohlenmonoxid befähigt ist, und in Gegenwart eines Lösungsmittels, dadurch gekennzeichnet, daß das Losungsmittels ein Keton ist, wobei die Hydroformulierungsreaktion 2-Hydroxy-tetrahydrofuran produziert.

2. Verfahren nach Anspruch 1, dadurch gekennzeichnet, aß das Keton-Lösungsmittel Acetophenon oder 2-Undecanon ist.

3. Verfahren nach Anspruch 1 oder 2, dadurch gekennzeichnet, daß die Temperatur im Bereiche von 50 bis 120°C und der Druck im Bereich von 3,5 bis 10,5 MPa ist.

4. Verfahren nach einem der Ansprüche 1 bis 3, dadurch gekennzeichnet, daß der Katalysator ein Rhodiumcarbonyl-triphenylphosphin-Komplex mit Überschuß an Triphenylphosphin ist.

5. Verfahren nach einem der Ansprüche 1 bis 3, dadurch gekennzeichnet, daß der Rhodiumcarbonyl-Katalysator ein Hydidocarbonyltris(triphenylphosphin)rhodium(I) in Gegenwart von Überschuß an Triphenylphospin ist.

**Revendications**

1. Procédé d'hydroformylation de l'alcool allylique consistant à faire réagir de l'alcool allylique avec un monoxyde de carbone et de l'hydrogène à une température supérieure à 25°C et sous une pression supérieure à 0,75 MPa en présence d'un catalyseur comprenant un rhodium carbonyle ou un composé de rhodium capable de former un rhodium-carbonyle en présence de monoxyde de carbone et en présence d'un solvant, caractérisé en ce que le solvant est une cétone et que la réaction d'hydroformulation donne du 2-hydroxytétrahydrofurane.

2. Procédé selon la revendication 1, caráctérisé en ce que le solvant cétonique est de l'acétophénone ou de la 2-undécanone.

3. Procédé selon la revendication 1 ou 2, caractérisé en ce que la température est choisie de 50 à 120°C et la pression de 3,5 à 10,5 MPa.

4. Procédé selon l'une des revendications 1 à 3, caractérisé en ce que le catalyseur est un complexe de rhodium et de carbonyl-triphénylphosphine avec un excès de triphénylphosphine.

5. Procédé selon l'une des revendications 1 à 3, caractérisé en ce que le catalyseur au rhodium-carbonyle est de l'hydridocarbonyltris(triphénylphosphine) rhodium (I) en présence d'un excès de triphénylphosphine.